# EUROPEAN PATENT SPECIFICATION

(11) **EP 1 213 001 B1**
(45) Date of publication and mention of the grant of the patent: **04.03.2009**
(21) Application number: 01850192.4
(22) Date of filing: 08.11.2001
(51) Int. Cl.: A61F 13/551, A61F 13/15

(54) **A sanitary napkin provided with a protective layer that functions as a protective wrapper**
Damenbinde mit einer Schutzschicht die als Verpackungsschicht dient
Serviette hygiénique avec une couche de protection fonctionnant comme emballage protecteur

(30) Priority: 08.12.2000 SE 0004537
(43) Date of publication of application: 12.06.2002
(73) Proprietor: SCA Hygiene Products AB, 405 03 Göteborg (SE)
(72) Inventor: Drevik, Solgun, 435 35 Mölnlycke (SE)
(74) Representative: Hyltner, Jan-Olof

(56) References cited:
- DE-U- 20 008 466
- GB-A- 2 306 428
- US-A- 5 037 417

## Description

### FIELD OF INVENTION

The present invention relates to an absorbent article in the form of a sanitary napkin, a panty liner or an incontinence napkin provided with a rectangular sheet which is folded double and which in its folded state embraces the absorbent article, wherein the mutually facing parts of the rectangular folded sheet are joined together so that said sheet will function as a protective wrapper for the absorbent article in its packaged state.

### BACKGROUND OF THE INVENTION

Sanitary napkins and similar absorbent articles are most often provided with one or more adhesive regions on their outer surface, so as to enable the article to be fastened to the inside of a pair of panties or underpants. Prior to use, the adhesive regions are covered with a protective layer to prevent the article from fastening to other objects and to protect the adhesive coatings from dust and like contaminants and also to prevent the adhesive from drying out. Sanitary napkins are also often folded into a packaging state. Folded sanitary napkins strive to return to their unfolded state and are often provided with a protective wrapper that prevents such unfolding of the napkin. The purpose of these protective wrappers is partly to facilitate packaging of several sanitary napkins in an outer packaging means, by keeping the napkins in their folded packaging state, and partly to keep the napkins in their folded packaged state in an outer package that has been opened, and partly to enable individual napkins to be stored temporarily in a hygienic fashion when removed from the outer packaging material, for instance in a handbag.

GB -A-2 273 279 teaches a sanitary napkin that is folded into a rectangular shape and provided with an adhesive coating protective layer that also functions as a single-piece packaging means by virtue of the fact that the edges of the protective layer folded one against the other are joined together.. This results in a protective wrapper that functions satisfactorily in respect of sanitary napkins that are folded into a rectangular shape, but not in respect of napkins that are folded into a triangular shape.

DE-U1-200 08 466 discloses a sanitary napkin according to the preamble of claim 1.

The object of the present invention is to provide an article which is folded into a triangular packaging state and which includes a protective wrapper that is able to keep the package-folded article in its folded state and which is also able to function as a protective layer for adhesive coatings on the outside of the article.

### SUMMARY OF THE INVENTION

This object is achieved in accordance with the invention by means of an absorbent article in the form of a sanitary napkin, a panty liner or an incontinence napkin that includes a rectangular sheet which is folded double and which in its folded state embraces the absorbent article, wherein the mutually facing or overlaid parts of the double-folded rectangular sheet are joined together so that the sheet will function as a protective wrapper for the absorbent article in its packaged state, characterised in that in its packaged state the absorbent article has a triangular shape with two edges that converge towards each other in a direction towards the pleat of the double-folded rectangular sheet; and in that the connection between the mutually facing, overlaid parts of the rectangular sheet include joins that extend along at least parts of the two mutually convergent edges of the packet-folded absorbent article. As a result of the joins between the mutually facing parts of the folded rectangular sheet extending along the side contours of the triangular folded article, the article is prevented from unfolding from its packaging state whilst at the same time ensuring that the article remains in its intended place in its protective wrapper. Moreover, this embodiment of an article protective wrapper can be readily produced. The joins also disclose the identity of the product, so that it can be readily distinguished from other rectangular packaged articles.

The invention also relates to an absorbent article in the form of a sanitary napkin, a panty liner or an incontinence napkin that includes a rectangular sheet which is folded double and embraces the absorbent article in its folded state, wherein the article is characterised in that an end-part of the rectangular sheet and an end-part of the article have been folded in against one another prior to double-folding the rectangular sheet, wherein the mutually facing or overlaid parts of the folded rectangular protective outer sheet are joined together so that said sheet will function as a protective wrapper for the absorbent article in its packaging state, and in that the absorbent article has, in its packaging state, a triangular shape that includes two edges which converge towards each other and towards the pleat of the double-folded rectangular sheet; and in that the connection between the mutually facing parts of the folded sheet includes joins that extend along at least parts of the two mutually convergent edges of the package-folded absorbent article.

In one preferred embodiment of the invention, the joins extend along the full length of the mutually converging edges of said article. The joins are rectilinear and may be continuous or discontinuous. The rectangular sheet may conveniently consist of a heat-weldable material and the joins may be seams produced by heat or ultrasound welding. Alternatively, glue joins may be used. The rectangular sheet may conveniently also consist of a protective layer for protecting an adhesive coating on the outside of the article.

### BRIEF DESCRIPTION OF THE DRAWINGS

The invention will now be described in more detail with reference to the accompanying drawings, in which
Fig. 1 illustrates schematically a first embodiment of an inventive sanitary napkin and shows the napkin in a non-folded state and laid on a rectangular sheet;
Fig. 2 shows the napkin of Figure 1 in its protective wrapper;
Fig. 3 shows a napkin according to a second embodiment of the invention in a non-folded state and laid on a rectangular sheet;
Fig. 4 shows the napkin of Figure 3 subsequent to having completed a first packaging folding stage; and
Fig. 5 shows the napkin of Figure 3 in its protective wrapper.

### DESCRIPTION OF EMBODIMENTS

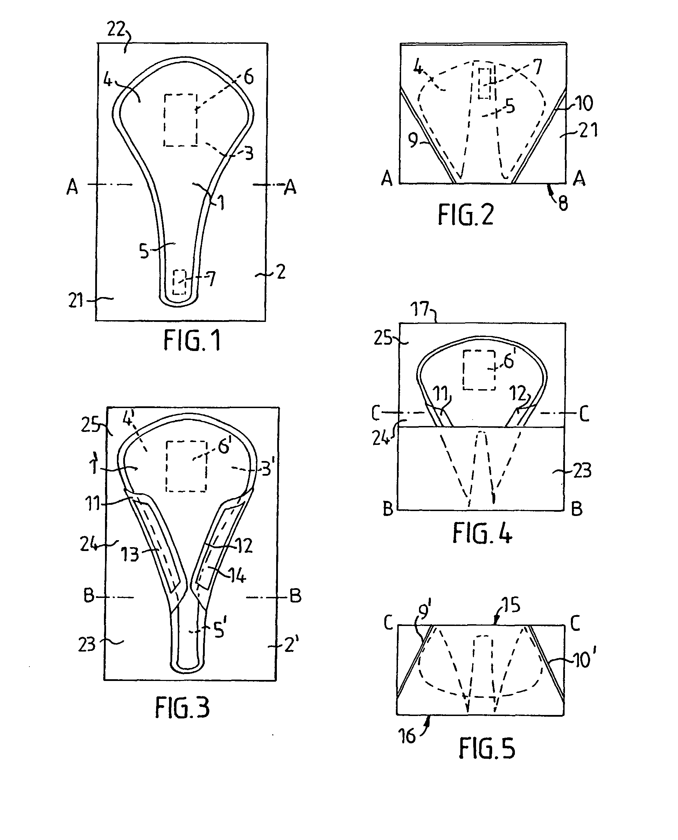

Figure 1 illustrates a sanitary napkin 1 placed on a rectangular sheet of material 2. The sheet 2 is longer and wider than the napkin 1 and extends beyond the napkin around the whole of its perimeter. The napkin typically includes an absorbent body 3 enclosed between a liquid-permeable and a liquid-impermeable outer sheet and has a front part 4 and a rear part 5. The napkin 1 also narrows rearwardly from a point of greatest width in its front part 4. The napkin 1 is intended to be fastened in the panties or underpants of the user and, to this end, is provided with adhesive coatings 6 and 7 disposed respectively on the front and rear parts of the liquid-impermeable outer sheet. These adhesive coatings 6, 7 are in abutment with the sheet 2 when the napkin 1 is placed thereon. In order to prevent the adhesiveness of the adhesive coatings from being impaired to any appreciable extent when removing the napkin from the sheet 2, the sheet is either produced from a material against which the adhesive coatings 6, 7 have poor adhesion, or is covered with a coating of such material within the region of respective adhesive coatings 6, 7.

It is, of course, possible to provide the adhesive coatings 6, 7 on the napkin 1 with separate protective layers, e.g. silicone-coated paper strips, so-called release paper, prior to placing the napkin on the sheet 2. However, this complicates the manufacture of the sanitary napkin and is therefore not preferred.

Subsequent to placing the napkin 1 on the sheet 2, the sheet 2 and the napkin 1 are folded about a fold axis A-A, such as to bring the lower half 21 of the sheet 2 in towards its upper half 22. As will be evident from Figure 2, which shows the sheet 2 and the napkin 1 subsequent to having been folded in this manner, the napkin obtains essentially the shape of a truncated triangle with the apex directed towards the pleat 8 obtained as a result of the double-fold.

After double-folding of the sheet 2 and the napkin 1, the mutually facing halves 21, 22 of the folded sheet 2 are joined together by joins 9, 10 which extend along the side edges of the double-folded napkin 1 that converge towards the pleat 8. The sheet 2 therewith forms a protective wrapper for the napkin 1, which has been folded double into a triangular shape.

Two purposes are achieved by allowing the joins to extend in this way. Firstly, the folded triangular napkin 1 is held in place in the wrapper, which would not be the case if the folded halves 21, 22 were to have been joined together along joins which extend along edges of said halves that extend at right angles to the pleat 8. Secondly, the napkin retains its folded shape. This would not be the case if the joins were to extend at right angles to the pleat 8, along the edges of the mutually facing, folded sheet-halves 21, 22, as the tendency of the napkin to return to its non-folded shape would not then be counteracted to any great extent by the walls of the wrapping formed by said sheet 2. The fact that the joins 9, 10 follow the side-edges of the folded napkin ensures that the napkin will be held securely in its intended place in the wrapper formed by the sheet 2, even when the outer packaging material is opened, whilst the wrapped napkin retains its folded state.

The joins 9, 10 may be continuous or discontinuous, for instance in the form of a row of punctiform joins. It may also be suitable to provide edge joins along the edges of the mutually facing, folded sheet-halves 21, 22 that extend perpendicular to the pleat 8, particularly when the joins 9, 10 solely consist of a few punctiform joins, partly to prevent the uppermost part of the napkin (as seen in the Figures) being exposed by unintentional down-folding of the uppermost parts of the sheet-halves 21, 22 when the joins 9, 10 terminate below the uppermost part of said napkin, and partly for aesthetic reasons.

The sheet 2 may be comprised of plastic and is provided with a plurality of outstanding projections, at least within the regions of the adhesive coatings 6, 7, so as to obtain a small contact surface area between adhesive and plastic sheet. Alternatively, the sheet may be comprised of paper that is coated with silicone in the regions of the adhesive areas of the napkin. Other alternative materials are waxed paper and patterned non-woven material or a non-woven and plastic film laminate as described in SE-C2-508 253. EP-A1-0 652 102 and GB-A-2 273 279 describe other known materials that can be used with respect to the sheet 2.

The joins may be seam welds when the sheet 2 is comprised of heat weldable material. Alternatively, the join may be glue joins. When the joins are glue joins, strings of glue are disposed on the sheet-half 22 prior to folding double the sheet 2. Seam welds, on the other hand, are made after folding double the sheet 2. The weld seams are conveniently produced by heat welding or ultrasound welding.

The adhesive coatings 6, 7 have been shown merely by way of example, and may be disposed in other ways. For instance, the adhesive region 6 may comprise three or more, continuous or discontinuous, parallel or offset, separate strings of adhesive or of other adhesive patterns. Correspondingly, similar regions that have low adhesiveness to the adhesive on the sheet 2 may be disposed in corresponding patterns or may be given a large surface area such as to cover more or all adhesive regions. When the sheet 2 is comprised of plastic film provided with outstanding projections, the projections may be disposed over the entire sheet.

Figure 1 shows the napkin placed on the sheet 2 in a non-folded configuration. Naturally, it is possible to place a sanitary napkin that is partially folded for packaging reasons on the sheet 2 prior to folding the sheet upon itself, i.e. double fold, even though this is not preferred.

Figures 3-5 illustrate a second embodiment of the invention. The difference between the sanitary napkin 1 shown in Figures 1 and 2 and the sanitary napkin 1' shown in Figures 3-4 is that the napkin 1' includes fastener flaps 11, 12 which are intended to be folded in against the outside of the wearer's panties or underpants and fastened thereto when in use. The sheet 2', on the other hand, is similar to the sheet 2 of the embodiment of Figures 1 and 2. Those components of the embodiment shown in Figures 3-4 that find correspondence with the components of the embodiment according to Figures 1 and 2 have been identified by the same reference signs as those used in Figures 1 and 2 but with the addition of a prime.

The sanitary napkin 1' has two fastener flaps 11, 12 which in the illustrated case are folded-in against the inside of the napkin 1', i.e. against the liquid-permeable outer sheet, and are provided with adhesive coatings covered by release paper 13, 14 or the like. The fastener flaps 11, 12 may equally as well be folded against the outside of the napkin 1', in which case the release paper may be omitted. However, it is preferred in this latter case to include release paper on the fastener flaps in order to facilitate handling of the napkin after having removed it from its wrapper. The napkin is placed on a sheet 2', which is similar to the aforedescribed sheet 2, at the beginning of the packaging process.

In a first packaging/folding stage, a first part 23 of the sheet 2' and the napkin 1' lying on said first part are folded onto remaining parts 24, 25 of the sheet 2' around a fold axis B-B. The napkin 1' and the sheet 2' have the configuration shown in Figure 4 after this folding stage.

The part 25 of the sheet 2' is then folded in against the parts 23, 24 about the axis C-C. Figure 5 shows the configuration of the napkin 1' and the sheet 2' after this latter folding stage. As will be apparent from Figure 5, the fully folded napkin 1' includes joins 9', 10' that extend along the side contours of the folded napkin, said side contours converging towards each other in a direction towards the pleat 15 formed by folding the napkin about the axis C-C.

The part 23 of the sheet 2' constitutes at most one-third of said sheet and in the case of the example shown in Figures 3-5 is somewhat smaller than a third. In the case of the illustrated example, the sheet parts 24, 25 are of mutually the same size, so that the edge of the inwardly folded part 25 will lie flush with the pleat 16 formed in the first folding stage about the axis B-B. However, this need not necessarily be the case since the part 25 may be smaller than the part 24, so that the edge 17 will be spaced from the pleat 16 subsequent to the final in-folding stage. However, the size of the parts 23-25 in relation to each other will be such that a portion of the part 25 will overlap the inwardly folded part 24 subsequent to folding about the axis C-C.

By a package folded napkin of triangular shape is meant in the present text all shapes of a sanitary napkin in which the side edges of said napkin extend obliquely in relation to a pleat of a package-folded sheet that forms a protective wrapper. The side edges of the package-folded napkin need not extend rectilinearly, and arched convergent lines are also included. Furthermore, those joins which follow the arched side edges of a triangular, package-folded napkin may be rectilinear provided that they follow the mean gradient of such edges towards a pleat formed by package-folding the sheet 2, 2'.

In the illustrated embodiments, the sheets 2 and 2' also form protective sheets for the adhesive coatings 6, 7 and 6', although said coatings may, of course, be protected with separate protective layers prior to being placed on the sheets 2 and 2'.

## Claims

1. An absorbent article (1) in the form of a sanitary napkin, a panty liner or an incontinence napkin, that includes a rectangular sheet (2) which is folded double and which in its folded state embraces the absorbent article, wherein the mutually facing or overlaid parts (21, 22) of the folded rectangular sheet are joined together so that the sheet will function as a protective wrapper for the absorbent article in its packaged state, the absorbent article (1) has in its packaged state a triangular shape that includes two edges that are mutually convergent in a direction towards the pleat (8) in the double-folded rectangular sheet (2); **characterised in that** the connection between said two mutually facing parts of the folded rectangular sheet includes joins (9, 10) that extend along at least parts of the two mutually convergent edges of the package-folded absorbent article.

2. An absorbent article (1') according to claim 1, **characterised in that** prior to said double folding, a first end-part (23) of the rectangular sheet (2') and an end-part of the article (1') lying on said end part (23) of the rectangular sheet (2) are together folded onto remaining parts (24, 25) of the sheet (2') around a first folding axis (B-B) and the second end part (25) is double folded onto the folded first end part (23) around a second fold axis (C-C).

3. An absorbent article according to Claim 1 or 2, **characterised in that** the joins (9, 10; 9', 10') extend along the full length of the mutually convergent edges of said article (1; 1').

4. An absorbent article according to Claim 1, 2 or 3, **characterised in that** the joins (9, 10; 9', 10') are continuous.

5. An absorbent article according to Claim 1, 2 or 3, **characterised in that** the joins (9, 10; 9', 10') are discontinuous.

6. An absorbent article according to any one of Claims 1-5, **characterised in that** the joins (9, 10; 9', 10') are rectilinear.

7. An absorbent article according to any one of Claims 1-6, **characterised in that** the rectangular sheet (2, 2') is comprised of heat-weldable material.

8. An absorbent article according to Claim 7, **characterised in that** the joins (9, 10; 9', 10') are seam welds produced by heat welding or ultrasound welding.

9. An absorbent article according to any one of Claims 1-7, **characterised in that** the joins (9, 10; 9', 10') are glue joins.

10. An absorbent article according to any one of the preceding Claims, **characterised in that** said rectangular sheet (2; 2') forms a protective layer for adhesive coatings (6, 7; 6') on the outside of the article (1; 1').

## Patentansprüche

1. Absorbensartikel (1) in der Form einer Binde, einer Slipeinlage oder einer Inkontinenzwindel, der einen rechteckigen Bogen (2) enthält, der doppelt gefaltet ist und der in seinem gefalteten Zustand den Absorbensartikel umspannt, wobei die gegenseitig zugewandten oder aufeinander gelegten Teile (21, 22) des gefalteten rechteckigen Bogens miteinander verbunden sind, so dass der Bogen als eine Schutzhülle für den Absorbansartikel in seinem verpackten Zustand fungiert, wobei der Absorbensartikel (1) in seinem verpackten Zustand eine dreieckige Gestalt hat, die zwei Kanten enthält, die zueinander konvergent in einer Richtung auf die Falte (8) in dem doppelt gefalteten rechteckigen Bogen (2) zu sind, **dadurch gekennzeichnet, dass** die Ankopplung zwischen zwei der gegenseitig zugewandten Teile des gefalteten rechteckigen Bogens Verbindungen (9, 10) enthält, die sich entlang zumindest von Teilen der zwei zueinander konvergenten Kanten des verpackt gefalteten Absorbensartikels erstrecken.

2. Absorbensartikel (1') nach Anspruch 1, **dadurch gekennzeichnet, dass** vor dem Doppelfalten ein erster Endteil (23) des rechteckigen Bogens (2') und ein Endteil des Artikels (1'), der auf dem Endteil (23) des rechteckigen Bogens (2) liegt, aufeinander auf verbleibende Teile (24, 25) des Bogens (2') um eine erste Faltachse (B-B) gefaltet sind, und der zweite Endteil (25) auf den gefalteten ersten Endteil (23) um eine zweite Faltachse (C-C) gefaltet ist.

3. Absorbensartikel nach Anspruch 1 oder 2, **dadurch gekennzeichnet, dass** sich die Verbindungen (9, 10; 9', 10') entlang der vollen Länge der zueinander konvergenten Kanten des Artikels (1; 1') erstrecken.

4. Absorbensartikel nach Anspruch 1, 2 oder 3, **dadurch gekennzeichnet, dass** die Verbindungen (9, 10; 9', 10') durchgehend sind.

5. Absorbensartikel nach Anspruch 1, 2 oder 3, **dadurch gekennzeichnet, dass** die Verbindungen (9, 10; 9', 10') unterbrochen sind.

6. Absorbensartikel nach einem der Ansprüche 1 bis 5, **dadurch gekennzeichnet, dass** die Verbindungen (9, 10; 9', 10') geradlinig sind.

7. Absorbensartikel nach einem der Ansprüche 1 bis 6, **dadurch gekennzeichnet, dass** der rechteckige Bogen (2, 2') aus heißschweißbarem Material besteht.

8. Absorbensartikel nach Anspruch 7, **dadurch gekennzeichnet, dass** die Verbindungen (9, 10; 9', 10') Schweißnähte sind, die durch Heißschweißen oder Ultraschallschweißen erzeugt sind.

9. Absorbensartikel nach einem der Ansprüche 1 bis 7, **dadurch gekennzeichnet, dass** die Verbindungen (9, 10; 9', 10') Klebeverbindungen sind.

10. Absorbensartikel nach einem der vorstehenden Ansprüche, **dadurch gekennzeichnet, dass** der rechteckige Bogen (2; 2') eine Schutzschicht'für Klebebeschichtungen (6, 7; 6') auf der Außenseite des Artikels (1; 1') bildet.

## Revendications

1. Article absorbant (1) sous la forme d'une serviette hygiénique, d'un protège-slip ou d'une serviette pour incontinence contenant une feuille rectangulaire (2) pliée en deux et qui, lorsqu'elle est pliée, entoure l'article absorbant, dans lequel les parties se trouvant face à face ou superposées (21, 22) de la feuille rectangulaire pliée sont jointes, de manière que la feuille forme une enveloppe protectrice pour l'article absorbant dans son état conditionné, l'article absorbant (1), dans son état conditionné, a une forme triangulaire comportant deux bords qui convergent mutuellement dans une direction vers le pli (8) de la feuille rectangulaire (2) pliée en deux, **caractérisé en ce que** la connexion entre lesdites deux parties se trouvant face à face de la feuille rectangulaire pliée comporte des jointures (9, 10) s'étendant sur au moins des parties des deux bords mutuellement convergents de l'article absorbant replié pour le conditionnement.

2. Article absorbant (1') selon la revendication 1, **caractérisé en ce que** avant ledit pliage en deux, une première partie terminale (23) de la feuille rectangulaire (2') et une partie terminale de l'article (1') reposant sur ladite partie terminale (23) de la feuille rectangulaire (2) sont repliées ensemble sur les parties résiduelles (24, 25) de la feuille (2') autour d'un premier axe de pliage (B-B) et la seconde partie terminale (25) est pliée en deux sur la première partie terminale pliée (23) autour d'un second axe de pliage (C-C).

3. Article absorbant selon la revendication 1 ou la revendication 2, **caractérisé en ce que** les jointures (9, 10; 9', 10') s'étendent sur toute la longueur des bords mutuellement convergents dudit article (1 ; 1').

4. Article absorbant selon la revendication 1, la revendication 2 ou la revendication 3, **caractérisé en ce que** les jointures (9, 10; 9', 10') sont continues.

5. Article absorbant selon la revendication 1, la revendication 2 ou la revendication 3, **caractérisé en ce que** les jointures (9, 10; 9', 10') sont discontinues.

6. Article absorbant selon l'une quelconque des revendications 1 à 5, **caractérisé en ce que** les jointures (9, 10 ; 9', 10') sont rectilignes.

7. Article absorbant selon l'une quelconque des revendications 1 à 6, **caractérisé en ce que** la feuille rectangulaire (2, 2') est constituée d'un matériau thermosoudable.

8. Article absorbant, selon la revendication 7, **caractérisé en ce que** les jointures (9, 10 ; 9', 10') sont des joints de soudure obtenus par thermosoudage ou par soudage par ultrasons.

9. Article absorbant selon l'une quelconque des revendications 1 à 7, **caractérisé en ce que** les jointures (9, 10 ; 9', 10') sont des joints de colle.

10. Article absorbant selon l'une quelconque des revendications précédentes, **caractérisé en ce que** ladite feuille rectangulaire (2 ; 2') constitue une couche protectrice pour les revêtements adhésifs (6, 7 ; 6') se trouvant à l'extérieur de l'article (1 ; 1').
